(19) Europäisches Patentamt
European Patent Office
Office européen des brevets

(11) Numéro de publication: 0 254 646
A1

# (12) DEMANDE DE BREVET EUROPEEN

(21) Numéro de dépôt: **87401710.6**

(22) Date de dépôt: **22.07.87**

(51) Int. Cl.[4]: **C 07 H 21/04**
C 12 Q 1/68

(30) Priorité: **22.07.86 FR 8610630**

(43) Date de publication de la demande:
**27.01.88 Bulletin 88/04**

(84) Etats contractants désignés:
**AT BE CH DE ES FR GB GR IT LI LU NL SE**

(71) Demandeur: **INSTITUT PASTEUR**
**25-28, rue du Docteur Roux**
**F-75724 Paris Cédex 15 (FR)**

(72) Inventeur: **Huynh Dinh, Tam**
**32 rue Paul Déroulède**
**F-78290 Croissy Sur Seine (FR)**

**Sarfati, Simon**
**142 rue de la Roquette**
**F-75011 Paris (FR)**

**Igolen, Jean**
**4 rue Croix Mathurine**
**F-78320 Le Mesnil Saint-Denis (FR)**

**Guesdon, Jean-Luc**
**12 rue du Hameau**
**F-75015 Paris (FR)**

(74) Mandataire: **Peaucelle, Chantal et al**
**S.C. Ernest Gutmann - Yves Plasseraud 67, boulevard Haussmann**
**F-75008 Paris (FR)**

## (54) Sondes de détection d'acides nucléiques renfermant des dérivés de désoxy-2' adénosine.

(57) Les sondes de l'invention sont élaborées à partir de dérivés d'adénosine répondant à la formule (I)

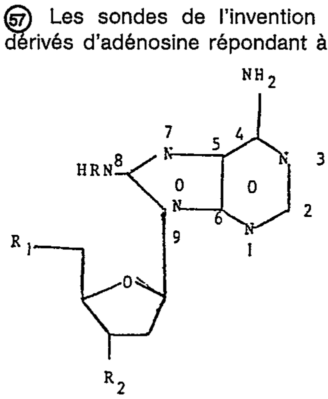

(I)

dans laquelle :

- R1 est choisi parmi le groupe -OH, $-OPO_3H_2$, $-OP_2O_6H_3$, $-OP_3O_9H_4$, ou un oligonucléotide,
- R2 est choisi parmi un atome d'hydrogène, un groupe -OH, un oligo- ou un polynucléotide,
- R représente une chaîne aminoalcoyle.

Ces sondes permettent la détection des séquences nucléotidiques complémentaires dans des échantillons biologiques.

EP 0 254 646 A1

**Description**

SONDES DE DETECTION D'ACIDES NUCLEIQUES RENFERMANT DES DERIVES DE DESOXY-2' ADENOSINE.

L'invention a pour objet des sondes de détection d'acides nucléiques renfermant des dérivés de désoxy-2' adénosine.

Il est maintenant bien connu des spécialistes d'avoir recours pour la détection et l'isolement des séquences d'ADN ou d'ARN, à des techniques basées sur l'hybridation entre une séquence déterminée de nucléotides utilisée en tant que sonde, et une séquence de nucléotides complémentaires de ceux de la sonde, éventuellement contenus dans une composition renfermant des acides nucléiques.

De manière classique, la détection de fragments d'ADN complémentaire (ADN-c) ou d'ARN messager (ARN-m) a été basée sur l'hybridation de ces fragments avec des oligodésoxynucléotides marqués avec des radioisotopes, principalement un isotope du phosphore ($^{32}P$).

De telles sondes marquées radioactivement et appelées de manière courante sondes chaudes, permettent une grande sensibilité de détection. Toutefois, l'utilisation de marqueurs radioactifs est limitée en raison de différents inconvénients.

D'une part, la durée de vie généralement courte des éléments radioactifs utilisés nécessite constamment le renouvellement des sondes, ce qui entraîne un prix de revient élevé.

D'autre part, l'emploi de substances radioactives demande un équipement spécialisé et des autorisations administratives quelquefois difficiles à obtenir.

De plus, l'automatisation des dosages réalisés avec de telles sondes chaudes s'avère difficile à mettre en pratique.

Récemment, des travaux ont été développés pour construire par voie enzymatique des sondes ne comportant pas d'éléments radioactifs. De telles sondes communément appelées sondes froides permettent la détection de l'ADN ou de l'ARN par réaction enzymatique ou immuno-enzymatique.

Il s'agit par exemple dans l'article de VINCENT C.et al, Nucleic Acids Res. 1982, 10, page 6787, de sondes froides basées sur le système antigène-anticorps monoclonaux(1,2), ou le système biotine-avidine (3) couplés avec une enzyme telle que la peroxydase, la phosphatase alcaline ou la galactosidase (3,4,5).

Depuis 1985, on a proposé des nucléotides modifiés en vue de leur incorporation et de leur détection enzymatique. Parmi ces nucléotides, on citera des dérivés de phosphoramidites biotinylés en 5', (6,7), des dérivés avec la photobiotine (8) et des nucléotides 5'-amino (9,10) qui peuvent être aussi bien incorporés dans les sondes froides que servir pour le séquençage d'ADN.

Dans la demande de brevet FR nº 84 13095, déposée le 22.08.1984, au nom des Demandeurs, on a décrit également des sondes constituées de fragments d'oligonucléotides comportant, en tant que base purique, des groupes adénines modifiés.

La poursuite des travaux réalisés par les inventeurs dans ce domaine leur ont permis d'élaborer des dérivés pouvant être incorporés par voie enzymatique dans des séquences d'oligonucléotides.

Ces dérivés de désoxy-2' adénosine permettent de mettre à profit les avantages des bases modifiées déjà mises au point, tout en disposant de produits possédant des propriétés améliorées.

Un procédé pour leur obtention est également décrit.

L'invention vise l'utilisation de ces dérivés pour la synthèse enzymatique de séquences d'oligonucléotides et l'utilisation de ces séquences en biologie, notamment pour l'élaboration de sondes de grande précision, stables dans le temps, avec lesquelles la détection est réalisée par des méthodes non radioactives faciles à utiliser.

Les dérivés de désoxy-2' adénosine comportent une chaîne aminoalcoyle en position 8 et répondent à la formule (I) :

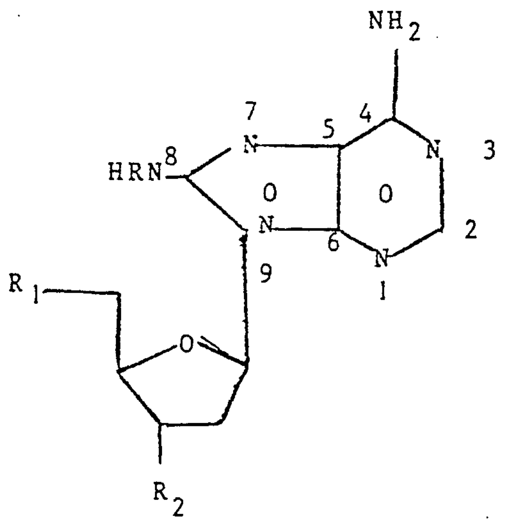

(I)

dans laquelle :
- $R_1$ est choisi parmi le groupe -OH, $-OPO_3H_2$, $-OP_2O_6H_3$, $-OP_3O_9H_4$, ou un oligonucléotide,
- $R_2$ est choisi un atome d'hydrogène, un groupe -OH, un oligo- ou un polynucléotide,
- R représente une chaîne aminoalcoyle.

Cette chaîne R répond plus spécialement à la structure $-(CH_2)_n$ - NHX, avec n représentant un entier de 4 à 12, X un atome d'hydrogène ou un groupe capable de servir de marqueur pour la molécule.

Des groupements marqueurs appropriés sont des groupements colorés ou fluorescents tels que la rhodamine, la fluorescéine ou le dansyl.

D'autres groupements X comprennent des haptènes.

D'autres groupements X encore sont formés par des enzymes, telles que la phosphatase ou la peroxydase.

Les peptides sont également utilisables en tant que groupement marqueur X.

D'une manière avantageuse, X représente la biotine.

Les dérivés définis ci-dessus permettent d'élaborer des sondes ayant une stabilité et une sensibilité accrues. Ils présentent également l'avantage de pouvoir être détectés par des méthodes immunoenzymatiques.

Selon un autre aspect de grand intérêt, ces nucléotides peuvent être incorporés par voie enzymatique (par exemple avec une polymérase, terminal-nucléotidyl-transférase, ligase) dans un fragment d'ADN ou d'ARN.

Les dérivés dans lesquels le groupement X de la chaîne de substitution aminoalcoyle représente un atome d'hydrogène permettent avantageusement l'accrochage avant ou après incorporation dans une séquence nucléotidique sur un groups marqueur.

les dérivés dans lesquels le groupe X est un groupe marqueur permettent, dans des essais d'hybridation avec des oligonucléotides renfermant de tels complémentaires de l'oligonucléotide, la détection des séquences sans marquage radio-actif.

Avantageusement, X est choisi parmi des groupements colorés ou fluorescents, notamment la rhodamine, la fluorescéine, le dansyl.

En variante, X est un haptène par exemple le dinitrophényle.

Dans d'autres dérivés avantageux, X représente une enzyme, notamment une peroxydase, une phosphatase.

Dans un autre mode de réalisation, X représente la biotine.

Les dérivés d'adénosine définis ci-dessus peuvent être incorporés enzymatiquement dans une chaîne nucléotidique par l'intermédiaire de $R_1$ et d'un groupe -OH en position 3' du motif sucre.

Les oligo ou polynucléotides résultants entrent également dans le cadre de l'invention.

Les dérivés d'adénosine ci-dessus sont avantageusement obtenus par voie de synthèse en utilisant un procédé comprenant la réaction d'un dérivé de désoxy-2' adénosine dont la position 8 de la base purique est activée, avec une diamine de formule (II) :

$NH_2-(CH_2)_n-NHX$ (II)

dans laquelle n et X sont tels que définis ci-dessus.

Comme dérivé d'adénosine activé sur la position 8, on utilise avantageusement un halogénure. Il s'agit plus spécialement d'un bromure ou encore d'un chlorure.

La réaction avec la diamine de formule (II) est réalisée en présence d'un solvant organique. Des solvants appropriés comprennent le méthanol, l'éthanol, le propanol, la pyridine.

Les dérivés d'adénosine dans lesquels la position 5' du motif sucre comporte un groupe phosphate, sont obtenus de préférence par phosphorylation avec un agent tel que le cyano-éthyl-phosphate, soit directement sur le nucléoside lorsque X représente un atome d'hydrogène, soit après blocage de la fonction amine libre.

Un blocage approprié est réalisé avec le groupe benzyloxycarbonyle.

Les dérivés di- et triphosphates correspondants sont obtenus par action de dérivé 5′-morpholidate avec un sel de phosphate ou de pyrophosphate.

Les nucléotides ci-dessus peuvent être incorporés enzymatiquement dans un oligonucléotide.

Avantageusement, cette incorporation est effectuée par la technique dite de "nick-translation".

Cette procédure permet l'incorporation uniforme de nucléotides dans un fragment d'ADN ou d'ARN, à des sites espacés régulièrement, en traitant ledit fragment d'ADN ou d'ARN avec une enzyme, notamment l'ADNase I d'E.coli, qui agit en fournissant des extrémités libres 3′-OH. La réaction avec l'ADN polymérase I permet ensuite l'incorporation des nucléotides souhaités dans le fragment d'ADN ou d'ARN.

En variante, l'incorporation enzymatique des nucléotides peut être réalisée par l'utilisation de la terminaldéoxytransférase avec un fragment d'ADN simple ou double brin.

Selon un autre mode de réalisation utilisant des dérivés préférés définis ci-dessus dans lesquels R représente une chaîne $-(CH_2)_n NH_2$, avec n tel que défini ci-dessus, on incorpore enzymatiquement le dérivé d'adénosine dans une séquence nucléotidique , puis on introduit chimiquement la biotine par action de l'ester N-hydroxy-succinimide de la biotine sur la séquence nucléotidique.

Les différents groupes X peuvent être introduits par réaction analogue.

Les dérivés d'adénosine ci-dessus constituent des intermédiaires de synthèse de grans intérêt pour l'élaboration de séquences de nucléotides utilisables comme sondes pour la détection et l'isolement d'une séquence d'acides nucléiques présents par exemple dans un mélange biologique liquide ou dans un lysat cellulaire.

Ces séquences de nucléotides, grâce aux modifications apportées au groupe adénine, sont capables de s'hybrider avec une sensibilité élevée avec des séquences complémentaires, formant des hybrides de grande stabilité.

Les sondes obtenues à partir de séquences de nucléotides renfermant les dérivés de l'invention présentent de plus une grande stabilité dans le temps, et peuvent être conservées pendant plusieurs années.

La détection des séquences nucléotidiques complémentaires s'hybridant avec la séquence nucléotidique contenue dans la sonde est réalisée par des méthodes non-radioactives basées sur le groupement X.

Par méthodes non-radioactives, on entend par exemple une réaction de détection par chimie luminescence, à l'aide de produits colorés ou fluorescents.

En variante, la détection peut être réalisée par une réaction immuno-enzymatique, utilisant des enzymes ou des anticorps.

Selon un mode de réalisation préféré de l'invention où le nucléotide est modifié par l'introduction d'une molécule de biotine, on réalise un couplage entre la biotine et une enzyme.

Avantageusement, on utilise l'avidine, qui forme avec la biotine un complexe stable. Ce couplage peut être effectué après la réaction d'hybridation.

Selon une variante de réalisation, l'avidine est couplée elle-même avec un produit de détection, par exemple, un composé coloré ou fluorescent, tel que la rhodamine ou la fluorescéine.

Selon une autre variante, l'avidine est couplée avec une enzyme formant, après réaction, des produits insolubles faciles à détecter.

Avantageusement, cette seconde enzyme est une phosphatase alcaline ou une peroxydase.

La détection de la biotine peut également être réalisée en utilisant un anticorps anti-biotine.

Il va de soi que cette modification chimique doit être telle qu'elle n'empêche pas l'hybridation ultérieure éventuelle de la sonde avec la séquence ou le fragment d'ADN recherché.

Les sondes de l'invention sont avantageusement utilisées pour détecter des séquences d'ADN complémentaires chez des bactéries et virus infectieux. Parmi ceux-ci, on citera Chlamydia, Campylobacter, Brucella, Parvovirus, le virus responsable de la rhizomanie de la betterave, le virus de l'hépatite B.

Ces sondes sont également utilisables pour détecter des résistances bactériennes aux antibiotiques.

D'autres caractéristiques et avantages de l'invention apparaîtront dans les exemples qui suivent relatifs à la synthèse de dérivés désoxy-2′ adénosine et à leur utilisation pout l'élaboration de sondes.

EXEMPLE DE SYNTHESE DE DERIVES PURIQUES SUBSTITUES EN POSITION 8 PAR UNE CHAINE BIOTINYLEE.

N-[amino-1-décanyl-10]-8-désoxy-2′ adénoisine (composé 1)

Un mélange (15,17 g, 47 mmoles) de bromo-8 désoxy-2′ adénosine (3) et (32 g, 186 mmoles) de diamino-1, 10 décane dans 350 ml d'éthanol est porté à reflux pendant 24 heures. La solution est ensuite concentrée à sec et le résidu obtenu lavé plusieurs fois à l'éther éthylique pout extraire la diamine en excès. Une fraction du résidu est purifiée sur colonne de silice sous légère pression utilisant, comme solvant le mélange tertiaire isopropanol-$NH_4OH$-$H_2O$, (6-1-1).

F: 190°C

Analyse Calculée pour $C_{20}H_{35}N_7O_3$ (421)

C 56,98; H 8,37; N 23,25

Analyse Trouvée C 56,83; H 8,59; N 23,65

N-[N-benzyloxycarbonylamino-1 décanyl-10]-8 désoxy-2′ adénosine (composé 2)

3,7 g (8,5 mmoles) de composé 1 sont dissous en chauffant dans un mélange de 30 ml d'eau et de 40 ml d'éthanol. A la solution revenue à la température ambiante, on ajoute (4,5 g, 17,7 mmoles) de chlorure de N-benzyloxy carbonyle N′-méthyle imidazolium et on maintient sous agitation magnétique pendant 24 heures. La solution est concentrée jusqu'à sécheresse et le sirop obtenu purifié par chromatographie sur silice ($CH_2Cl_2$-MeOH) (MeOH = méthanol).

On obtient 3,33 g de produit benzyloxycarbonylé correspondant à un rendement de 72%.

N-benzoyl-6 N-[N-benzyloxycarbonylamino-1 décanyl-10] -8 désoxy-2′ adénosine (composé 3)

2,38 g (4,28 mmoles) du composé 2 sont séchés par deux coévaporations avec de la pyridine (Merck) puis dissous dans 15 ml de ce même solvant anhydre.

A 0°C (bain: eau plus glace) on ajoute goutte à goutte 3,7 ml de chlorure de benzoyle et on laisse le mélange revenir à température ambiante ; après 24 heures on détruit l'excès de réactif par addition de 5 ml de méthanol et on concentre la solution à sec. Le résidu est dissous dans 100 ml de $CH_2Cl_2$ et lavé avec une solution saturée de $NaHCO_3$ puis à l'eau ; la phase organique est séchée sur sulfate de sodium, filtrée et évaporée sous vide.

Au sirop dissout dans un mélange de 12,6 ml de pyridine et 6,4 ml d'éthanol (refroidi dans un bain d'eau et de glace) on ajoute 9,5 ml de soude 8N. Après une demi-heure la solution est neutralisée à l'aide de résine Dowex 50 WX8 sous forme H+ ; la résine est filtrée, rincée au méthanol et le solvant est concentré jusqu'à sécheresse. Le dérivé monobenzoylé cristallise dans l'éthanol

F : 87-88°C (éthanol)

RMN $^1$H 400 MHz: ($CDCl_3$ + TMS) : δ = 1,20 (m, 16H, $CH_2$(3,8); 1,38 (m, 2H, $CH_2$(9); 1,55 (m, 2H, $CH_2$(2)); 2,22 (m, 1H, H″2); 2,67 (m, 1H, H′2); 3,10 (m, 2H, $CH_2$(10)); 3,35 (m, 2H, $CH_2$ (1)); 3,87 (m, 2H, H5′5″); 4,08 (m, 1H H′4); 4,69 (m, 1H, H′3); 4,76 (s, 1H, NH); 5,06 (s, 3H, $CH_2$φ + NH); 6,50 (q, 1H, H′1); 7,37 (s, 5H, φ ); 7,41-7,49 (m, 3H, H φ′p,H φ′mm′); 7,98-8,00 (s+s, 2H, Hφ′oo′); 8,4 (s, 1H,H2).

N-benzoyl-6 benzoyl-3′ N-[N-benzyloxycarbonylamino-1 décanyl-10]-8 désoxy-2′ adénosine (Composé 4)

A 2 g (3 mmoles) du composé 3 dissous dans 6.5 ml de pyridine, on ajoute 1,13 g (3,3 mmoles) de chlorure de diméthoxytrityle. Après 2 heures et demie à température ambiante, on détruit l'excès de réactif par addition de quelques gouttes de méthanol, on concentre à sec et on dissout le résidu dans du $CH_2Cl_2$, on lave avec une solution saturée de bicarbonate de sodium puis à l'eau. On sèche la phase organique sur sulfate de sodium. Après filtration et évaporation sous vide, on lave le précipité à l'éther de pétrole.

On dissout le dérivé tritylé (1,85 g, 1,9 mmole) dans 25 ml de pyridine (Merck) et on ajoute 2,6 g (11,4 mmoles) de chlorure de benzoyle et 115 mg de DMAP. Après une heure et demie à température ambiante, on concentre sous vide, on dilue avec du $CH_2Cl_2$, on lave avec une solution saturée de bicarbonate de sodium puis à l'eau et on précipite à l'éther de pétrole.

Le précipité est dissous dans 30 ml d'un mélange $CH_2Cl_2$-MeOH (7-3) contenant 2% de BSA; 10 minutes après et à température ambiante, la solution est lavée ($NaHCO_3$ et $H_2O$), séchée et concentrée sous vide. Le résidu est purifié par chromatographie sur silice (solvant $CH_2Cl_2$-MeOH).

RMN $^1$H 400 MHz: ($CDCl_3$ + TMS) : δ = 1,25 (m, 16H, $CH_2$ (3-8)); 1,46 (m, 2H, $CH_2$ (9)); 1,63 (m, 2H, $CH_2$(2)); 2,47 (dd; 1H, H″2); 2,97 (m, 1H, H′2); 3,17 (q, 2H, $CH_2$ (10)), 3,47 (q, 2H, $CH_2$ (1)); 4,03 (d, 1H, H″5); 4,16 (d, 1H, H′5); 4,31 (m, 1H, H′4); 5,08 (s, 2H, $CH_2$φ); 5,71 (d, 1H, H′3); 6,66 (q, 1H, H′1); 7,33 (s, 5H, φ); 7,45-7,65 (m, 2H, Hφ′mm′); 7,82 (d, 1H, Hφ′p); 8,00-8,12 (2d, 2H, Hφ′oo′); 8,57 (s, 1H, $H_2$); 9,10 (s, 1H, NHCOφ′).

N-[amino-1 décanyl-10]-8 désoxy-2′ triphosphate-5′ adénosine (composé 5)

Une solution de 327 mg (0,428 mmole) du composé 4, 1 mmole de cyano éthylphsophate sel de pyridinium (1 ml d'une solution stock à 1 mmole/ml) dans 5 ml de pyridine est concentrée sous vide à 30°C; le sirop obtenu est dissous à deux reprises dans 10 ml de pyridine puis évaporé à sec ; on recommence la même opération avec de la pyridine anhydre.

Le résidu sec est dissout dans 5 ml de pyridine anhydre, on ajoute environ 0,5 g (6 eq.) de N, N′-dicyclohexylcarbodiimide (DCC) et on laisse le mélange à température ambiante 48 heures sous agitation magnétique. On ajoute 3 ml d'eau, on laisse sous agitation pendant une heure, on chasse la pyridine à sec et on reprend le résidu avec 10 ml d'eau; on filtre la dicyclohexylurée formée et on la rince avec 10 ml d'eau. On concentre le filtrat et on reprend le résidu dans 30 ml d'$NH_4OH$ 6N et on l'incube 24 heures à 60°C; on concentre la solution ammoniacale et on purifie le sirop obtenu sur Sephadex G10 utilisant comme solvant le mélange éthanol-eau, (EtOH-$H_2O$), (1-2). Après lyophilisation des fractions positives en UV et au dosage du phosphore on obtient 178 mg (rendement 49%) de monophosphate.

200 mg de monophosphate sont dissous dans un mélange eau-méthanol (1-1) puis la solution est passée sur une colonne (3 x 1 cm) de résidu Dowex 50 WX8 sous forme morpholinium; la colonne est lavée avec ce mélange jusqu'à ce que l'effluent n'absorbe plus dans l'UV.

La solution est concentrée; on reprend le résidu dans 3 ml d'alcool terbutylique et 3 ml $H_2O$, on ajoute 105 µl de morpholine distillée (4 eq.) et, goutte à goutte, 259 mg (1,25 mmole) de DCC dissous dans 4,5 ml d'alcool terbutylique. On porte à reflux pendant 4 heures. Une chromatographie sur couche mince de silice, utilisant comme solvant le mélange isopropanol-$NH_4OH$-$H_2O$ (7-1-2) indique que la réaction est terminée.

La solution est refroidie à température ambiante, on filtre le précipité formé que l'on lave à l'alcool terbutylique; le filtrat est évaporé à sec. Le résidu est dissous dans l'eau et la phase aqueuse extraite 3 fois à l'éther (15 ml). La concentration de la phase aqueuse conduit au morpholidate avec un rendement de 75%.

A 178 mg (1 mmole) d'acide pyrophosphorique dissous dans 5 ml d'eau, on ajoute 10 ml de pyridine (Merck) et 1,0 ml (4,2 mmoles) de tri-n-butylamine distillée. Cette solution homogène est évaporée jusqu'à obtention d'un sirop que l'on déshydrate par 4 coévaporations successives avec 10 ml de pyridine anhydre.

La pyridine résiduelle est ensuite éliminée par deux coévaporations avec du toluène (5 ml) fraîchement distillé et recueilli sur tamis moléculaire 4°A.

Le pyrophosphate sel de tri-n-butylamine est ensuite rajouté au morpholidate dissous dans du diméthyle sulfoxyde (DMSO) (5 ml, anhydre).

A la solution maintenue à température ambiante on ajoute après 24 heures, 0,5 mmole du sel de tri-n-butylamine de l'acide pyrophosphorique préparé comme précédemment. Après 3 jours à température ambiante, le morpholidate a disparu, on ajoute 5 ml d'eau et l'on dépose la solution sur une colonne (2,5 x 30 cm) de DEAE cellulose sous forme bicarbonate. La colonne est lavée à l'eau jusqu'à ce que l'effluent n'absorbe plus dans l'UV puis éluée avec un gradient linéaire 0-0,35 M de bicarbonate de triéthylammonium.

Les fractions (3 ml) contenant le triphosphate identifié (UV - phosphore) sont réunies et évaporées à sec (bain-marie 30-35°C); le TEAB est éliminé après plusieurs évaporations avec du méthanol, plusieurs dissolutions dans l'eau suivies de lyophilisations.

A partir de 200 mg de monophosphate, 36 mg de triphosphate ont été obtenus, soit un rendement de 23%.

Spectrométrie de masse

Théorique $C_{20}H_{38}N_7O_{12}P_3$ M = 661,16

Trouvé $[M + H]^+$ = 662,16

RMN $^1H$ 400MHz: ($D_2O$): $\delta$ = 1,8 (m, 14H, decyl protons); 1,49 (t, 2H, decyl protons); 1,61 (m, 2H, decyl protons); 2,18 (dd, 2H, H"2); 2,68 (m, 1H, H'2); 2,84 (t, 2H, decyl protons); 3,38 (m, 2H, H5'5"); 4,14 (dd, 1H, H'4); 4,30 (t, 1H, H'3); 4,65 (HDO); 6,35 (q, 1H, H'1); 7,98 (s, 1H, H2).

RMN $^{31}P$ 162 MHz: ($D_2O$):par rapport à $PO_4H_3$ = - 8,209 (d, 1P, $P_\gamma$, $J_{P\gamma\text{-}P\beta}$ = 19,45 Hz); - 9,866 (d, 1P, $P_\alpha$, $J_{P\alpha}$, $P_\beta$, $J_{P\alpha}$ $P_\beta$ = 19,45 Hz, $J_{P\alpha\text{-}P\beta}$ = 0 HZ); - 20,987 (t, 1P, $P_\beta$, $J_{P\beta\text{-}P\alpha}$ = $J_{P\beta}$ - $P_\gamma$ + 19,45 Hz).

N-[N-biotinyl-1 décanyl-10]-8 désoxy-2' triphosphate-5' adénosine (composé 6)

Au triphosphate 5 (9 mg) on ajoute une solution de bicarbonate de sodium 1 M (200 µl) et de l'ester de la N-hydroxy succinimide de la biotine (5 mg) dissous dans du diméthylformamide (DMF) (100 µl fraîchement distillé.

Après 24 heures à 4°C (test ninhydrine: négatif) on ajoute de l'eau (1 ml) au milieu réactionnel puis on le dispose sur une colonne de chromatographie Séphadex G10. L'élution à l'eau fournit essentiellement deux pics absorbant dans l'ultraviolet, seul l'un d'eux donne une réponse positive au test des sucres et négative au test des amines; les fractions de ce dernier (3 ml) sont réunies et évaporées à sec puis purifiées en chromatographie à phase liquide à haute performance (CLHP) (colonne Nucléosil 5C18 4,6 x 250 mm) et lyophilisées. On obtient un rendement de 16%.

Spectroscopie de masse

théorique $C_{30}H_{52}N_9O_{14}SP_3$ M = 887

trouvé $[M + H]^+$ = 888,63

On donne ci-après un exemple de détection d'une séquence d'ADN réalisée avec des dérivés préférés de l'invention.

Détection d'une séquence nucléotidique d'ADN comportant une partie du génome du virus de l'hépatite B

I. MATERIELS ET METHODES

Le composé 6 est incorporé enzymatiquement par la technique de "nick-translation" dans le plasmide pCP10 (12) contenant deux fois les parties du génome du virus de l'hépatite B.

L'incorporation enzymatique a été réalisée en présence de dCTP tritié afin de pouvoir suivre indirectement le taux de substitution.

Le composé 6 constitue un substrat utilisable pour l'ADN polymérase I de E. coli.

En présence de 0,2 mM de composé 6, 14 % des adénosines ont été substitués dans 300 ng du plasmide pCP10.

La détermination du seuil de détection de cette sonde biotinylée a été calculée en utilisant la technique suivante :

Un microlitre de différentes concentrations de la sonde biotinylée est déposé sur une membrane de nitrocellulose. Cette membrane est ensuite cuite pendant 2 heures à 80°C sous vide, saturée en présence de tampon tris-HCl 10 mM, pH 8, contenant du chlorure de sodium (0,15 M) de l'albumine bovine (1 %) et du Tween (0,1 %) afin de diminuer les adsorptions non spécifiques. La détection de la sonde immobilisée sur la membrane est réalisée par une incubation d'1 heure avec de l'avidine, de la streptavidine ou des anticorps anti-biotine marqués par la phosphate alcaline, suivie d'une incubation de 30 minutes dans le substrat approprié. Quelque soit le système de détection (avidine, streptavidine ou anticorps) et dans les conditions

utilisées il a été possible de détecter jusqu'à 1,5 pg de sonde.

L'utilisation de cette sonde biotinylée pour détecter l'ADN cible contenant une séquence d'ADN homologue, montre que la limite de détection est égale à 8 pg en suivant le protocole classique d'hybridation sous forme simple brin à 68°C pendant 16 heures avec la sonde concentrée à 75 ng/ml (13).

La sonde biotinylée peut également être utilisée è 42°C avec 50 % de formamide. Elle est parfaitement stable dans ces conditions.

Ainsi qu'il a été déjà dit et comme il résulte d'ailleurs de ce qui précède, l'invention ne se limite nullement aux exemples de réalisation qui ont été plus particulièrement décrits mais en embrasse au contraire toutes les variantes.

BIBLIOGRAPHIE

1. Methods in enzymology, 92, 47

2. C. VINCENT, P. TCHEN, M. COHEN-SOLAL et P. KOURILSKY, Nucleic Acids Res. (1982), 10, 6787

3. D. C. WARD et coll. Proc. Natl. Acad. Sci. USA (1981), 78, 6633 ; (1983), 80 4045

4. M. RENZ et C. KURTZ, Nucleic Acids Res. (1984), 12, 3435

5. A. MURASUGI et R.B. WALLACE DNA (1984), 3(3), 269

6. A. CHOLLET et E.H. KAWASHIMA Nucleic Acids Res. (1985), 13, 1529

7. T. KEMPE, W.I. SUNQUIST, F. CHOW et S.L HU, Nucleic Acids Res. (1985), 13, 4

8. A.C FOSTER, J.L. Mc INVES, D.C. SKINGLE et R.H. SYMONS, Nucleic Acids Res. (1985), 13, 745

9. L.M. SMITH, S. FUNG, M.N. HUNKAPILLER, T.J. HUNKAPILLER et L.E. HOOD, Nucleic Acids Res. (1985), 13, 2399

10. Biotechnology (1985), 3, 395

11. M. IKEHARA et M. KANEDO, Chem. Pharm. Bull. (1970), 18, 2441.

12. DUBOIS M.F., POURCELL C., ROUSSET S., CHANY C., THIOLLAIS P., Proc. Natl. Acad. Sci. USA (1980), 77, 45-53

13. MANIATIS T., FRITSCH F., SAMBROOK J., Molecular Cloning, Lab. Manual Cold Spring Harbor Laboratory (1982), p. 387

**Revendications**

1. Sondes de détection d'acides nucléiques, caractérisées en ce qu'elles sont élaborées à partir de dérivés de désoxy-2' adénosine répondant à la formule I

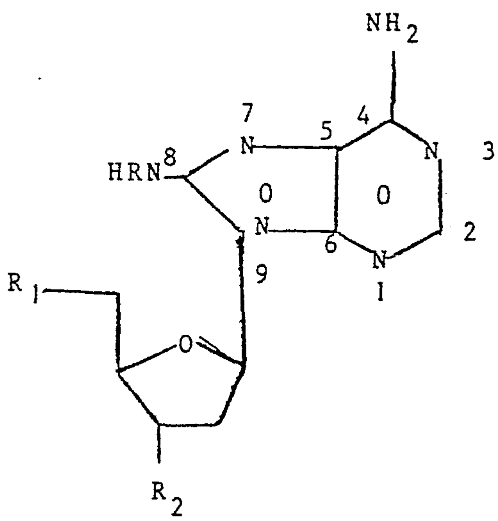

(I)

dans laquelle :

- R1 est choisi parmi le groupe -OH, $-OPO_3H_2$, $-OP_2O_6H_3$, $-OP_3O_9H_4$, ou un oligonucléotide,

- R2 est choisi parmi un atome d'hydrogène, un groupe -OH, un oligo- ou un polynucléotide,
- R représente une chaîne aminoalcoyle.

2. Sondes selon la revendication 1, caractérisées en ce que la chaîne R répond à la structure $-(CH_2)_n$ -NHX, avec n représentant un entier 4 à 12, X un atome d'hydrogène ou un groupe capable de servir de marqueur pour la molécule.

3. Sondes selon la revendication 2, caractérisées en ce que le groupe marqueur est choisi dans les groupements colorés ou fluorescents tels que la rhodamine, la fluorescéine ou le dansyl, les haptènes, des enzymes telles que la phosphatase ou la peroxydase, des peptides ou de la biotine.

4. Sondes selon l'une quelconque des revendications 1 à 3, caractérisées en ce que les motifs adénosine sont incorporés dans la chaîne nucléotidique par voie enzymatique.

Office européen des brevets

# RAPPORT DE RECHERCHE EUROPEENNE

Numéro de la demande

EP 87 40 1710

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| X | WO-A-8 403 285 (MOLECULAR BIOSYSTEMS, INC.)<br>* Pages 25-29,65; revendications * | 1-3 | C 07 H 21/04<br>C 12 Q 1/68 |
| A | WO-A-8 302 277 (INSTITUT PASTEUR)<br>* Pages 10-13 * | 1-3 | |
| A | WO-A-8 302 276 (INSTITUT PASTEUR)<br>* Pages 10,11 * | 1-3 | |
| D,A | FR-A-2 569 407 (INSTITUT PASTEUR)<br>* Revendications * | 1-3 | |
| | | | **DOMAINES TECHNIQUES RECHERCHES (Int. Cl.4)** |
| | | | C 07 H 21/00<br>C 07 H 19/00 |

Le présent rapport de recherche a été établi pour toutes les revendications

| Lieu de la recherche | Date d'achèvement de la recherche | Examinateur |
|---|---|---|
| LA HAYE | 21-10-1987 | VAN BIJLEN H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82